# DEMANDE DE BREVET EUROPEEN

(11) **EP 1 891 925 A1**
(43) Date de publication de la demande: **27.02.2008**
(21) Numéro de dépôt: 07112087.7
(22) Date de dépôt: 09.07.2007
(51) Int. Cl.: A61K 8/04, A61K 8/37, A61K 8/39

(54) **Composition cosmétique huileuse en aérosol**

(30) Priorité: 25.07.2006 FR 0653094
(71) Demandeur: L'Oréal, 75008 Paris (FR)
(72) Inventeur: Aubrun-Sonneville, Odile, 92160, Antony (FR); Bordeaux, Dominique, 91450, Soisy sur Seine (FR)
(74) Mandataire: Rasson, Catherine

(57) **Abrégé**

L'invention a pour objet un produit aérosol comprenant une composition huileuse contenant au moins une huile, au moins un composé hydrocarboné ayant un point de fusion supérieur ou égal à 30°C, le dit composé se trouvant sous forme de particules solides, la quantité de composé(s) hydrocarboné(s) allant de 0,5 à 20 % en poids par rapport au poids total de la composition huileuse, la quantité d'eau et de composés hydrosolubles étant inférieure à 15 % en poids par rapport au poids total de la composition huileuse, et
(b) un ou plusieurs gaz propulseurs.
Le composé hydrocarboné peut être notamment une paraffine ou un amide d'acide gras.

Le produit est notamment utilisé pour le nettoyage et/ou le démaquillage de la peau et/ou des cheveux, le soin de la peau et/ou des cheveux, pour la protection de la peau contre le soleil, pour le maquillage de la peau.

## Description

L'invention a pour objet un produit aérosol comprenant une composition cosmétique huileuse apte à donner une mousse stable, contenant un composé se trouvant sous forme de particules solides, et l'utilisation du dit produit sous forme d'aérosol pour le nettoyage et/ou le démaquillage de la peau et/ou des cheveux, pour le soin de la peau et/ou des cheveux, pour la protection de la peau contre le soleil, pour le maquillage de la peau.

Les huiles sont très largement utilisées dans le domaine cosmétique, certaines d'entre elles pour leur efficacité démaquillante, d'autres pour leurs propriétés nutritives et protectrices de la peau ou du cheveu. Ces huiles sont très souvent formulées dans des émulsions. Toutefois, pour maximaliser leurs effets tant pour le démaquillage que pour la nutrition, elles peuvent constituer un support cosmétique à part entière, par exemple quand on les utilise comme huiles démaquillantes rinçables, comme huiles corporelles pour le traitement des peaux sèches, comme huiles capillaires pour le traitement des cheveux secs, comme produits de maquillage, ou comme huiles solaires.

Leur application sur la peau n'est en général pas très aisée du fait de leur fluidité qui rend leur manipulation difficile. Par ailleurs, la sensation grasse qu'elles procurent est souvent perçue comme négative. Il peut donc être avantageux de les présenter sous forme de mousse, car cela conduit à un produit structuré plus facile à manipuler, et la perception sensorielle est nettement améliorée du fait que le produit est plus moelleux, puisqu'il se présente sous la forme d'une mousse à l'application, minimisant la perception de gras.

Les huiles donnant une mousse peuvent être obtenues par pressurisation d'huile avec un gaz propulseur et conditionnement en aérosol. La difficulté est d'obtenir des mousses qui soient fines tout en présentant une stabilité suffisante.

Ainsi, le document EP-A-1,438,946 décrit des compositions pour aérosol délivrant une mousse d'huile, comprenant un corps gras ou une huile et un ester gras de polyglycérol. Le document JP-A-04/89424 décrit une composition sous forme d'aérosol comprenant une huile, une zéolite, un tensioactif, un agent moussant et un propulseur. Le document JP-A-09/110636 décrit une composition moussante de nettoyage à base d'huile comprenant un propulseur et un liquide comprenant des huiles, un ester gras de dextrine, et un tensioactif non ionique. Le document US-A-2005/186147 décrit une composition moussante contenant des particules solides d'oxyde métallique, de silice ou de métaux, un solvant hydrophobe, de l'eau, un tensioactif, un stabilisant/agent gélifiant, et un gaz propulseur. Toutefois, les mousses décrites selon ces documents s'avèrent grossières et/ou fugaces, « fugaces » signifiant un temps de stabilité de la mousse, inférieur à 1 minute.

Il subsiste donc le besoin de disposer d'une composition huileuse moussante en aérosol, qui soit confortable et qui donne une mousse stable, c'est-à-dire une mousse qui subsiste de préférence au moins 5 minutes et mieux au moins 10 minutes.

Or, la demanderesse a découvert de manière surprenante que les composés hydrocarbonés se trouvant sous forme de particules solides dans la composition huileuse permettaient d'atteindre le but recherché et d'obtenir une composition huileuse qui, utilisée en aérosol, donne une mousse stable et fine, agréable à utiliser.

La présente invention a donc pour objet un produit aérosol pour application topique comprenant :
(a) une composition huileuse contenant au moins une huile et au moins un composé hydrocarboné ayant un point de fusion supérieur ou égal à 30°C, le dit composé se trouvant sous forme de particules solides, la quantité de composé(s) hydrocarboné(s) allant de 0,5 à 15 % en poids par rapport au poids total de la composition, la quantité d'eau et de composés hydrosolubles étant inférieure à 15 % en poids par rapport au poids total de la composition huileuse, et
(b) un ou plusieurs gaz propulseurs.

On entend par « produit aérosol » un produit conditionné dans un dispositif aérosol, adapté à la pressurisation. Ce dispositif comprend généralement un flacon pressurisé muni d'une tête de distribution et il est apte à distribuer une mousse quand on actionne la tête de distribution.

Le produit aérosol selon l'invention est destiné à une application topique et contient donc un milieu physiologiquement acceptable. Par milieu physiologiquement acceptable, on entend un milieu compatible avec la peau, les muqueuses (y compris l'intérieur des paupières, les lèvres), les ongles, la zone oculaire et/ou les fibres kératiniques (cheveux et cils). Ce produit constitue notamment une composition cosmétique ou dermatologique.

La composition huileuse (a) est généralement anhydre, c'est-à-dire exempte d'eau et de composés hydrosolubles tels que les polyols et les alcools monohydriques en C2-C6. Toutefois, elle peut contenir de l'eau et des composés hydrosolubles à condition que la quantité totale d'eau et de composés hydrosolubles soit d'au plus 15 % en poids et de préférence d'au plus 10 % en poids par rapport au poids total de la composition. Le teneur en quantité totale d'eau et de composés hydrosolubles peut donc aller de 0 à 15 % et de préférence de 0 à 10 % du poids total de la composition. On entend par « polyols » des composés hydrosolubles contenant au moins deux fonctions hydroxyle, tels que par exemple la glycérine, le propylène glycol, le butylène glycol, l'isoprène glycol et le PEG-8. On entend par « alcools monohydriques en C2-C6 » des composés contenant une seule fonction hydroxyle, tels que par exemple l'éthanol ou l'isopropanol. De préférence, la composition huileuse est exempte d'eau.

Le produit aérosol peut contenir une quantité de composition huileuse allant par exemple de 80 à 97% en poids, de préférence de 80 à 95 % en poids et mieux de 85 à 95 % en poids par rapport au poids total du produit.

La composition huileuse utilisée en aérosol mousse à la sortie de l'aérosol, et elle peut donc être appelée aussi composition moussante, du fait qu'elle forme une mousse quand on l'utilise en aérosol contenant un gaz propulseur.

La mousse obtenue à la sortie de l'aérosol selon l'invention présente l'avantage d'être fine et stable, cette stabilité étant due à la présence des particules solides de composé hydrocarboné dans la composition introduite dans l'aérosol.

L'invention a encore pour objet une mousse obtenue par pulvérisation du produit aérosol tel que défini ci-dessus.

### Composés hydrocarbonés

On entend par « composé hydrocarboné », un composé comportant majoritairement des atomes de carbone et d'hydrogène. Ces composés ne sont pas des polymères, les polymères étant définis comme étant des composés contenant plusieurs fois la même unité et ayant un poids moléculaire important, alors que les composés hydrocarbonés selon l'invention sont des composés non polymériques et ils ont généralement un poids moléculaire inférieur à 1000, et mieux inférieur à 500.

Selon un mode particulier de réalisation de l'invention, le composé hydrocarboné utilisé contient au moins un hétéroatome. Ce ou ces hétéroatomes présents dans ce composé peuvent être notamment l'azote et/ou l'oxygène. Le composé hydrocarboné peut donc contenir un ou plusieurs atomes d'azote ou un ou plusieurs atomes d'oxygène ou à la fois des atomes d'azote et d'oxygène.

Les composés hydrocarbonés utilisés dans la composition de l'invention ont un point de fusion supérieur ou égal à 30°C et se présentent sous forme de particules solides dans la composition de l'invention, c'est-à-dire que, dans la composition qui est à une température inférieure à leur point de fusion, ils ne sont pas solubilisés dans les huiles mais ils restent sous forme solide. Toutefois, ce sont des composés hydrophobes, qui sont solubles dans les huiles à une température supérieure à leur point de fusion. Les composés hydrocarbonés utilisés ont généralement un point de fusion allant de 30°C à 200°C, mieux de 30°C à 150°C, et encore mieux de 30 °C à 100°C.

Les composés hydrocarbonés peuvent être choisis en particulier parmi :
- les paraffines ;
- les alcools gras qui contiennent de l'oxygène comme hétéroatome;
- les acides gras qui contiennent de l'oxygène comme hétéroatome ;
- les amides d'acide gras qui contiennent de l'oxygène et de l'azote comme hétéroatomes;
et les mélanges de ces composés.

On entend dans la présente demande par « acide gras », les acides comportant au moins 12 atomes de carbone, de préférence de 12 à 30 atomes de carbone, plus particulièrement de 14 à 22 atomes de carbone, et par « alcool gras », les alcools comportant de 12 à 30 atomes de carbone, plus particulièrement de 14 à 22 atomes de carbone.

Comme alcool gras, on peut citer par exemple l'alcool cétylique, mais il s'agit de préférence des alcools en C30 à C50, comme le produit commercialisé par la société New Phase Technologies sous la dénomination Performacol 550-L-Alcohol (nom INCI : C30-C50 Alcohols).

Comme acide gras, on peut citer par exemple l'acide palmitique.

Comme paraffine, on peut citer plus particulièrement la paraffine raffinée, notamment celle commercialisée sous la dénomination Cerafine 56/58 par la société Sasol.

Comme amides d'acides gras, on peut citer les amides dérivés d'un acide comportant au moins 12 atomes de carbone. Ces amides sont donc des amides d'acide comportant au moins 12 atomes de carbone, notamment des amides d'acide comportant de 12 à 30 atomes de carbone, plus particulièrement de 14 à 22 atomes de carbone. Le terme « amide » inclut les amides et leurs dérivés tels que par exemple les dérivés comportant une ou plusieurs fonctions hydroxylées, comme par exemple les éthanolamides (monoéthanolamides = MEA, ou diéthanolamides = DEA), les mono-isopropanolamides (MIPA), et tout autre dérivé d'amide.

On peut citer notamment les amides suivants (en nom INCl) :
- Cocamide MEA (monoéthanolamide d'acides de coprah),
- Cocamide MIPA (monoisopropanolamide d'acides de coprah),
- Cocamide DEA (diéthanolamide d'acides de coprah),
- Stearamide MEA (monoéthanolamide d'acide stéarique),
- Hydroxystearamide MEA (N-(2-hydroxyethyl)-12-hydroxystearamide),
- Myristamide MEA (monoéthanolamide d'acide myristique);
- Lauramide MEA (monoéthanolamide d'acide laurique),
- Palmitamide MEA (monoéthanolamide d'acide palmitique),
- Myristoyl/Palmitoyl Oxostearamide/Arachamide MEA,
- Cetyl-PG Hydroxyethyl Decanamide,
- Cetyl-PG Hydroxyethyl Palmitamide,
- Dibutyl Lauroyl Glutamide,
- Ethylene Distearamide,
- Hydroxycetyl Hydroxyethylstearamide,
- PEG-4 Stearamide.
- et leurs mélanges.

De préférence, les composés hydrocarbonés sont choisis parmi les paraffines, les alcools gras en C30-C50, les amides d'acide gras, et leurs mélanges. Plus préférentiellement, ils sont choisis parmi les amides d'acide comportant au moins 12 atomes de carbone, notamment les amides cités ci-dessus.

Les composés hydrocarbonés utilisés selon l'invention peuvent être présents en une quantité allant de 0,5 à 15 % en poids, de préférence de 1 à 15 % en poids, mieux de 1 à 10 % en poids et encore mieux de 2 à 10 % en poids par rapport au poids total de la composition huileuse.

### Huiles

La composition huileuse de l'invention comporte une ou plusieurs huiles, et la quantité d'huiles peut aller par exemple de 60 à 99 % en poids, de préférence 65 à 98 % en poids, mieux de 70 à 98 % en poids et encore mieux de 75 à 98 % en poids par rapport au poids total de la composition huileuse. On entend par « huile » un corps gras liquide à la température ambiante (20à25°C).

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 22 atomes de carbone comme les triglycérides des acides heptanoïque ou octanoïque ou encore, par exemple les huiles d'amande douce, de maïs, de soja, de courge, de coriandre, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'isopropyle, le palmitate d'éthyl-2-hexyle (ou palmitate d'octyle), le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrityle ; l'ester d'acide benzoïque et d'alcools en C12-C15 (nom INCl : C12-15 Alkyl Benzoate) ;
- les hydrocarbures linéaires ou ramifiés de point de fusion inférieur à 30°C, d'origine minérale ou synthétique, tels que les huiles de paraffine, volatiles ou non, et leurs dérivés, la vaseline, l'isohexadecane, l'isododecane, le polyisobutène hydrogéné tel que l'huile de Parléam® ; les alcanes cycliques tels que le dioctyl cyclohexane ;
- les alcools gras ayant de 8 à 26 atomes de carbone, de point de fusion inférieur à 30°C, tels que l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1^{®}" et "FLUTEC PC3^{®}" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluorométhoxybutane vendu sous la dénomination "MSX 4518^{®}" par la Société 3M et le nonafluoroéthoxyisobutane ; les dérivés de perfluoromorpholine, tels que la 4-trifluorométhyl perfluoromorpholine vendue sous la dénomination "PF 5052^{®}" par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par "huile hydrocarbonée" dans la liste des huiles citées ci-dessus, toute huile comportant majoritairement des atomes de carbone et d'hydrogène, et éventuellement des groupements ester, éther, fluoré, acide carboxylique, alcool, silicone, amine, phényl, et/ou aminoacide.

### Additifs

La composition huileuse du produit de l'invention peut également contenir différents additifs qui peuvent être solubles dans les huiles, ou être en dispersion dans le milieu huileux.

Ainsi, elle peut contenir par exemple un ou plusieurs tensioactifs solubles ou dispersibles dans les huiles.
Comme tensioactifs de ce type, on peut citer par exemple :
- les esters d'acide gras et de polyol oxyéthylénés, tels que le tri-iso-stéarate de glycéryle oxyéthyléné (20 OE) (nom INCI : PEG-20 glycéryl triisostéarate), le trioléate de sorbitane 20 OE (nom INCI : Polysorbate 85) ; les esters d'acides gras oxyéthylénés et leurs dérivés comme le mono-isostéarate de polyéthylène glycol (8 OE) (nom INCI : PEG-8 isostéarate) et le copolymère de polyéthylène glycol (30 OE) et d'acide 12-hydroxy stéarique (Arlacel P135 de la société Uniqema ; nom INCI : PEG-30 Dipolyhydroxystéarate);
- les tensioactifs siliconés tels que les alkyl diméthicone copolyols, notamment ceux ayant un radical alkyle comportant de 10 à 22 atomes de carbone et comportant de 2 à 50 groupes oxyéthylénés et de 2 à 50 groupes oxypropylénés, tel que le cétyl diméthicone copolyol (nom INCl: Cetyl PEG/PPG-10/1 Dimethicone) comme le produit commercialisé sous la dénomination Abil EM-90 par la société Goldschmidt ; le lauryl diméthicone copolyol (nom INCl : Lauryl PEG/PPG-18/18 Methicone) et par exemple le mélange d'environ 91% de lauryl diméthicone copolyol et d'environ 9 % d'alcool isostéarylique, commercialisé sous la dénomination DOW CORNING 5200 FORMULATION AID par la société Dow Corning ; les dimethicone copolyols, notamment parmi ceux comportant de 2 à 50 groupes oxyéthylénés et de 2 à 50 groupes oxypropylénés, comme par exemple celui comportant 18 groupes oxyéthylénés et 18 groupes oxypropylénés (PEG/PPG-18/18 Dimethicone), tels que le mélange de dimethicone copolyol comportant 18 groupes oxyéthylénés et 18 groupes oxypropylénés, de cyclopentasiloxane et d'eau (10/88/2), commercialisé par la société Dow Corning sous la dénomination DC-3225C ou DC2-5225C (nom INCl: Cyclopentasiloxane / PEG/PPG-18/18 Dimethicone), ou celui comportant 14 groupes oxyéthylénés et 14 groupes oxypropylénés (PEG/PPG-14/14 Dimethicone) tels que, notamment, le mélange de dimethicone copolyol comportant 14 groupes oxyéthylénés et 14 groupes oxypropylénés et de cyclopentasiloxane (85/15) commercialisé sous la dénomination Abil EM-97 par la société Goldschmidt (nom INCl : Bis-PEG/PPG-14/14 Dimethicone / Cyclopentasiloxane) ;
- les alkylpolyglucosides (APG) tels que le decylglucoside comme le produit commercialisé sous la dénomination MYDOL 10® par la société Kao Chemicals, le produit commercialisé sous la dénomination PLANTAREN 2000 UP® par la société Cognis, et le produit commercialisé sous la dénomination ORAMIX NS 10® par la société Seppic ; le caprylyl/capryl glucoside comme le produit commercialisé sous la dénomination ORAMIX CG 110® par la Société Seppic ; le laurylglucoside comme les produits commercialisés sous les dénominations PLANTAREN 1200 N® et PLANTACARE 1200® par la société Cognis ; et le coco-glucoside comme le produit commercialisé sous la dénomination PLANTACARE 818/UP® par la société Cognis ;
- et les mélanges de ces tensioactfifs.

Quand ils sont présents, la quantité de tensioactif(s) peut aller par exemple de 0,5 à 30 % en poids, mieux de 1 à 20 % en poids et encore mieux de 5 à 20 % en poids par rapport au poids total de la phase huileuse.

Selon un mode préféré de réalisation de l'invention, la composition huileuse (a) contient au moins un tensioactif, notamment un tensioactif non ionique choisi parmi les esters d'acide gras et de polyol oxyéthylénés.

La composition huileuse de l'invention peut contenir aussi des particules comme par exemple des pigments tels que le dioxyde de titane, les oxydes de zirconium, de cérium, de zinc ou de fer (noir, jaune ou rouge) ; des argiles telles que le disteardimonium hectorite ; des silices ; des particules contenant des copolymères acryliques (nom INCl : Acrylates Copolymer) telles que les micro-sphères: expancées de chlorure de vinylidène/acrylonitrile/PMMA, commercialisées sous les dénominations Expancel ; les particules d'amidons ; et leurs mélanges.

La composition huileuse de l'invention peut contenir également des actifs cosmétiques, comme par exemple les anti-oxydants, les agents kératolytiques et anti-âge comme les alpha-hydroxyacides (acide lactique, acide glycolique, acide citrique) et les bêta-hydroxyacides (acide salicylique et dérivés tels que les acides N-alkyl salicyliques, par exemple l'acide N-octanoyl-5 salicylique) ; les vitamines comme la vitamine E (tocophérol et dérivés), la vitamine C (acide ascorbique et dérivés), la vitamine A (rétinol et dérivés) ; les adoucissants ; les filtres UV organiques ou minéraux ; et tout actif habituellement utilisés selon le but d'utilisation de la composition.

En outre, la composition huileuse de l'invention peut contenir des polymères lipophiles comme les copolymères blocs dérivés du styrène comme le copolymère styrène/ethylene-butylene/styrene tel que le produit commercialisé sous la dénomination Kraton G-1650E par la société Kraton Polymers ; les copolymères d'acide acrylique ou méthacrylique, tels que le copolymère Acrylate/stearyl acrylate/dimethicone methacrylate commercialisé sous la dénomination KP 561 P par la société Shin Etsu ; les Poly-C10-30-Alkyl Acrylates comme le produit commercialisé sous la dénomination Interlimer IPA 13-1 par la société Landec ; les copolymères d'éthylènediamine/stearyl dimer dilinoleate, tels que le produit commercialisé sous la dénomination Uniclear 100 VG par la société Arizona Chemical.

Comme indiqué ci-dessus, la composition huileuse de l'invention peut également contenir des solvants hydrophiles comme l'eau, les polyols et les alcools monohydriques en C2-C6, en une quantité maximale de 15 % en poids et de préférence de 10 % en poids par rapport au poids total de la composition huileuse. Toutefois, elle est de préférence exempte d'eau

La présence d'alcool tel que l'éthanol peut être utile pour avoir une meilleure restitution du produit en fluidifiant les jus qui sont fortement gélifiées par la présence de composés hydrophobes de l'invention. Elle permet également d'améliorer la stabilité de certaines compositions qui ont tendance à épaissir dans le temps. Ainsi, la composition peut contenir de l'alcool et notamment de l'éthanol en une quantité allant par exemple de 0,01 à 10 % en poids, de préférence de 0,05 à 10 % en poids et mieux de 0,05 à 5 % en poids par rapport au poids total de la composition huileuse.

### Gaz propulseur

Le produit aérosol selon l'invention comporte la composition huileuse décrite ci-dessus et un ou plusieurs gaz propulseurs. Les gaz propulseurs utilisés dans le produit selon l'invention sont choisis parmi les gaz liquéfiés tels que par exemple le diméthyléther (DME) ; les hydrocarbures volatils tels que le n-butane, le propane, l'isobutane, et leurs mélanges, éventuellement avec au moins un hydrocarbure chloré et/ou fluoré, comme par exemple les composés vendus par la société Dupont de Nemours sous les dénominations Fréon® et Dymel®, et en particulier le monofluorotrichlorométhane, le difluorodichlorométhane, le tétrafluorodichloroéthane, et le 1,1-difluoroéthane vendu notamment sous la dénomination commerciale DYMEL 152 A par la société DUPONT. On peut y ajouter éventuellement un gaz comprimé tel que le gaz carbonique, le protoxyde d'azote, l'azote et/ou l'air.

La quantité de gaz liquéfié peut aller par exemple de 5 à 20% en poids et mieux de 5 à 15 % en poids par rapport au poids total du produit aérosol.

Le produit aérosol selon l'invention est mis dans un conditionnement adapté aux aérosols.

La pression dans l'aérosol peut varier de 1 à 10 bars et de préférence de 1,5 à 7 bars. Le ou les gaz comprimés qui peuvent être ajoutés au gaz liquéfié permettent d'atteindre une pression plus importante que si le gaz liquéfié était le seul gaz propulseur, et, quand ils sont présents, les gaz comprimés sont ajoutés en une quantité suffisante pour avoir la pression que l'on souhaite obtenir, par exemple une pression d'au moins 3 bars et de préférence d'au moins 5 bars.

Le produit aérosol selon l'invention peut être utilisé dans le domaine cosmétique notamment pour le nettoyage et/ou le démaquillage de la peau et/ou des cheveux, pour le soin de la peau et/ou des cheveux, pour la protection de la peau contre le soleil ou les UV, pour le maquillage de la peau, par exemple comme produit de nettoyage et/ou de démaquillage de la peau et/ou des cheveux, comme huile corporelle pour adoucir et nourrir la peau, comme huile solaire pour se protéger des rayons du soleil ou des rayons UV, ou comme huile capillaire pour protéger les fibres capillaires, les nourrir et faciliter le démêlage, comme produit de maquillage pour les cils ou la peau. La mousse formée est fine et stable.

Aussi, la présente demande a encore pour objet l'utilisation cosmétique du produit aérosol tel que défini ci-dessus, pour le nettoyage et/ou le démaquillage de la peau et/ou des cheveux, pour le soin de la peau et/ou des cheveux, pour la protection de la peau contre le soleil ou les U.V., pour le maquillage de la peau.

Les compositions huileuses du produit selon l'invention peuvent être préparées en mélangeant les constituants à une température supérieure ou égale à la température de fusion des composés hydrocarbonés solides, ce mélange se faisant de préférence à la turbine. Puis, les compositions sont refroidies sous agitation jusqu'à refroidissement à température ambiante (environ 20 à 25°C),. les composés hydrocarbonés solides se solidifiant au cours du refroidissement. Les compositions sont ensuite pressurisées en utilisant un ou plusieurs gaz propulseurs, et éventuellement un ou plusieurs gaz liquéfiés.

L'invention a encore pour objet un procédé de préparation du produit aérosol tel que défini ci-dessus, consistant à préparer une composition telle que définie ci-dessus, en mélangeant les constituants à une température supérieure ou égale à la température de fusion des composés hydrocarbonés, à refroidir la composition sous agitation jusqu'à refroidissement à température ambiante, à ajouter à la composition, un ou plusieurs gaz propulseurs (un ou plusieurs gaz liquéfiés et éventuellement un ou plusieurs gaz comprimés).

### Exemples

Les exemples suivants sont donnés à titre d'illustration de l'invention et n'ont pas de caractère limitatif. Toutes les quantités sont données en pourcentage en poids de matière première par rapport au poids total de la composition.

### Exemples 1 à 3 selon l'invention :

| Composition A (sans gaz) | Exemple 1 | Exemple 2 | Exemple 3 |
|---|---|---|---|
| Ethylhexyl palmitate | 52.45 | 50.45 | 37,5 |
| Isononyl isononanoate | 28 | - | 36,5 |
| Isododécane | - | 28 | |
| conservateurs | 0.55 | 0.55 | |
| PEG-20 glycéryl triisostéarate (1) | 16 | 8 | 16 |
| Cocamide MEA (2) | 3 | | |
| Cocamide MIPA (3) | - | 5 | |
| Paraffine (4) | - | - | 10 |
| | | | |

| Composition B = Aérosol (composition A + gaz) | | | |
|---|---|---|---|
| Composition A | 90 | 90 | 90 |
| Diméthyl éther | - | 10 | - |
| Isobutane | 10 | - | 10 |
| Azote sous une pression de 5 bars | Qsp jusqu'à 5 bars | Qsp jusqu'à 5 bars | Qsp jusqu'à 5 bars |
| Aspect du jus | Gel de particules, qui se fluidifie sous agitation | Suspension de particules | Suspension de particules |
| Qualité de la mousse | Mousse fine et stable | Mousse fine et stable | Mousse assez fine et stable |

| | | | |
|---|---|---|---|
| (1) EMALEX GWIS-320EX de la société Ajinomoto (2) COMPERLAN 100 de la société Cognis (3) REWOMID V 3203 de la société Goldschmidt (4) CERAFINE 56/58 PASTILLES de la société Sasol | | | |

Les exemples 1 et 3 peuvent constituer par exemple, des produits de nettoyage de la peau, et l'exemple 2 peut constituer par exemple une huile corporelle.

### Exemples comparatifs 1 à 3

| Composition A (sans gaz) | Exemple comparatif 1 | Exemple comparatif 2 | Exemple comparatif 3 |
|---|---|---|---|
| Ethylhexyl palmitate | 39.225 | 55.45 | - |
| Isononyl isononanoate | 39.225 | - | 75.45 |
| Isododécane | - | 28 | - |
| conservateurs | 0.55 | 0.55 | 0.55 |
| PEG-20 glycéryl triisostéarate (1) | 16 | 8 | 16 |
| Palmitate de dextrine (5) | 5 | - | - |
| | | | |
| Composition B = Aérosol (composition A + gaz) | | | |
| Compositon A | 90 | 90 | 90 |
| Diméthyl éther | 10 | - | 10 |
| isobutane | - | 10 | - |
| Azote comprimé sous 5 bars | Qsp jusqu'à 5 bars | - | - |
| Aspect du jus | Solution épaissie avec grumeaux | Solution transparente | Solution transparente |
| Qualité de la mousse | Mousse fine se déstabilisant rapidement | Mousse grossière et instable | Mousse très grossière et instable |

| | | | |
|---|---|---|---|
| (1) EMALEX GWIS-320EX de la société Ajinomoto (5) RHEOPEARL KL2 - OR de la société Chiba Flour Milling | | | |

Ces exemples comparatifs montrent que, en l'absence de composé formant des particules solides dans l'huile et ayant un point de fusion supérieur ou égal à 30°C, le jus peut être hétérogène (voir exemple comparatif 1) et la mousse obtenue est soit fine mais instable (exemple comparatif 1) soit grossière et instable (exemples comparatifs 2 et 3).

### Exemples comparatifs 4 et 5

| Composition A (sans gaz) | Exemple comparatif 4 | Exemple comparatif 5 |
|---|---|---|
| Ethylhexyl palmitate | 40 | 40 |
| Isononyl isononanoate | 39 | 39 |
| PEG-20 glycéryl triisostéarate (1) | 16 | 16 |
| Zeolite (6) | 5 | - |
| Polyméthyl Silsesquioxane (7) | - | 5 |
| | | |
| Composition B = Aérosol (composition A + gaz) | | |
| Composition A | 90 | 90 |
| isobutane | 10 | 10 |
| Azote comprimé sous 5 bars | Qsp jusqu'à 5 bars | Qsp jusqu'à 5 bars |
| Aspect des jus | Solution transparente avec dépôt solide au fond qui ne se disperse pas sous agitation | Solution transparente avec dépôt solide au fond qui ne se disperse pas sous agitation |
| Qualité de la mousse | Mousse très instable | Mousse très instable |

| | | |
|---|---|---|
| (1) EMALEX GWIS-320EX de la société Ajinomoto (6) X-MOL de Zeochem (7) TOSPEARL 145A de GE Toshiba Silicones | | |

Ces exemples comparatifs 4 et 5 montrent que le remplacement de composé formant des particules solides dans l'huile et ayant un point de fusion supérieur ou égal à 30°C, par des particules solides polymériques, donne un jus hétérogène, et une mousse d'aérosol instable.

### Exemples 4 à 6 selon l'invention

| Composition A | Exemple 4 | Exemple 5 | Exemple 6 |
|---|---|---|---|
| Ethylhexyl palmitate | 58.45 | 50.45 | 52.45 |
| Isononyl isononanoate | - | 28 | 28 |
| Isododécane | 28 | - | - |
| conservateurs | 0.55 | 0.55 | 0.55 |
| Ethanol | - | 2 | - |
| Polyglyceryl-4 Isostearate (5) | 8 | - | - |
| Polysorbate 85 (ou Trioléate de sorbitane 20 OE) (6) | - | - | 16 |
| PEG-20 glycéryl triisostéarate (1) | - | 16 | - |
| Cocamide MEA (2) | 2 | 3 | 3 |
| Cocamide MIPA (3) | 4 | - | - |
| | | | |
| Composition B = Aérosol (composition A + gaz) | | | |
| Composition A | 90 | 90 | 90 |
| Isobutane | - | 10 | 10 |
| Diméthyl éther | 10 | - | - |
| Azote sous une pression de 5 bars | - | - | Qsp jusqu'à 5 bars |
| Aspect du jus | Solution épaissie de particules | Solution épaissie de particules | Gel de partiicules |
| Qualité de la mousse | Mousse fine et stable | Mousse fine et stable | Mousse fine et stable |

| | | | |
|---|---|---|---|
| (1) EMALEX GWIS-320EX de la société Ajinomoto (2) COMPERLAN 100 de la société Cognis (3) REWOMID V 3203 de la société Goldschmidt (5) ISOLAN GI 34 de la société Goldschmidt (6) RHEODOL TW - 0320 V de la société KAO | | | |

L'exemple 4 peut constituer par exemple une huile corporelle, et les exemples 5 et 6 peuvent constituer par exemple des produits de nettoyage de la peau.

### Exemple 7 selon l'invention : fond de teint

| Composition A (sans gaz) | Exemple 7 |
|---|---|
| Ethylhexyl palmitate | Qsp 100 % |
| Isononyl isononanoate | 40 |
| Isododécane | - |
| conservateurs | 0.55 |
| PEG-20 glycéryl triisostéarate (1) | 4 |
| Cocamide MEA (2) | 3 |
| Oxyde de titane (anatase non traité) | 6 |
| Oxydes de fer brun, jaune | 1 |
| | |
| Composition B = Aérosol (composition A + gaz) | |
| Composition A | 90 |
| Isobutane | 10 |
| Azote sous une pression de 5 bars | Qsp jusqu'à 5 bars |

| | |
|---|---|
| (1) EMALEX GWIS-320EX de la société Ajinomoto (2) COMPERLAN 100 de la société Cognis | |

### Exemple 8 selon l'invention :: composition solaire

| Composition A | Exemple 8 |
|---|---|
| Ethylhexyl palmitate | Qsp 100 % |
| Isododécane | 28 |
| conservateurs | 0.55 |
| PEG-20 glycéryl triisostéarate (1) | 8 |
| Cocamide MEA (2) | 2 |
| Cocamide MIPA (3) | 4 |
| Butyl Methoxy Dibenzoylméthane (7) | 4 |
| Octocrylene (8) | 4 |
| Dioxyde de Titane (9) | 3,5 |
| Composition B = Aérosol (composition A + gaz) | |
| Composition A | 90 |
| Diméthyl éther | 10 |

| | |
|---|---|
| (1) EMALEX GWIS-320EX de la société Ajinomoto (2) COMPERLAN 100 de la société Cognis (3) REWOMID V 3203 de la société Goldschmidt (7) PARSOL 1789 de la société DSM Nutritional Products (8) UVINUL N539 de la société BASF (9) MICRO TITANIUM DIOXIDE MT100 AQ de la société TAYCA) | |

## Revendications

1. Produit aérosol pour application topique, comprenant (a) une composition huileuse contenant au moins une huile et au moins un composé hydrocarboné ayant un point de fusion supérieur ou égal à 30°C, le dit composé se trouvant sous forme de particules solides, la quantité de composé(s) hydrocarboné(s) allant de 0,5 à 15 % en poids par rapport au poids total de la composition, la quantité d'eau et de composés hydrosolubles étant inférieure à 15 % en poids par rapport au poids total de la composition huileuse, et
(b) un ou plusieurs gaz propulseurs.

2. Produit aérosol selon la revendication 1, **caractérisé en ce que** le composé hydrocarboné contient au moins un hétéroatome.

3. Produit aérosol selon la revendication précédente, **caractérisé en ce que** l'hétéroatome est l'azote et/ou l'oxygène.

4. Produit aérosol selon la revendication 1, **caractérisé en ce que** le composé hydrocarboné est choisi parmi les paraffines, les alcools gras, les acides gras, les amides d'acide gras, et leurs mélanges.

5. Produit aérosol selon la revendication précédente, **caractérisé en ce que** le composé hydrocarboné est un amide dérivé d'acide comportant au moins 12 atomes de carbone.

6. Produit aérosol selon la revendication précédente, **caractérisé en ce que** l'amide est choisi parmi le Cocamide MEA, le Cocamide MIPA, le Cocamide DEA, le stearamide MEA, l'Hydroxystearamide MEA, le Myristamide MEA, le Lauramide MEA, le Palmitamide MEA, le Myristoyl/palmitoyl oxostearamide/arachamide MEA, le Cetyl-PG hydroxyethyl Decanamide, le Cetyl-PG hydroxyethyl palmitamide, le Dibutyl lauroyl glutamide, l'Ethylene Distearamide, l'Hydroxycetyl hydroxyethylstearamide, le PEG-4 Stearamide, et leurs mélanges.

7. Produit aérosol selon la revendication 4, **caractérisé en ce que** l'alcool gras est choisi parmi les alcools gras en C30 à C50.

8. Produit aérosol selon la revendication 1, **caractérisé en ce que** le composé hydrocarboné est une paraffine raffinée.

9. Produit aérosol selon l'une quelconque des revendications précédentes, **caractérisé en ce que** la quantité d'huiles va de 60 à 99 % en poids par rapport au poids total de la composition huileuse.

10. Produit aérosol selon l'une quelconque des revendications précédentes, **caractérisé en ce que** la composition huileuse contient en outre un ou plusieurs tensioactifs solubles ou dispersibles dans les huiles.

11. Produit aérosol selon la revendication précédente, **caractérisé en ce que** le tensioactif est choisi parmi les esters d'acide gras et de polyol oxyéthylénés, les esters d'acides gras oxyéthylénés et leurs dérivés, les tensioactifs siliconés, les alkylpolyglucosides, et leurs mélanges.

12. Produit aérosol selon l'une quelconque des revendications précédentes, **caractérisé en ce que** la composition huileuse contient en outre au moins un alcool monohydrique en C2-C6.

13. Produit aérosol selon l'une quelconque des revendications précédentes, **caractérisé en ce que** la quantité de composition huileuse va de 80 à 97% en poids, de préférence de 80 à 95 % en poids par rapport au poids total du produit.

14. Produit aérosol selon l'une quelconque des revendications précédentes, **caractérisé en ce que** le gaz propulseur est un gaz liquéfié choisi parmi le diméthyléther, le n-butane, le propane, l'isobutane, et leurs mélanges.

15. Produit aérosol selon la revendication précédente, **caractérisé en ce que** la quantité de gaz liquéfié va de 5 à 20% en poids par rapport au poids total du produit.

16. Produit aérosol selon l'une quelconque des revendications précédentes, **caractérisé en ce qu'**il contient en outre un gaz comprimé choisi parmi le gaz carbonique, le protoxyde d'azote, l'azote ou l'air.

17. Produit aérosol selon l'une quelconque des revendications précédentes, **caractérisé en ce qu'**il est pressurisé sous une pression variant de 1 à 10 bars.

18. Utilisation cosmétique du produit aérosol selon l'une quelconque des revendications 1 à 17, pour le nettoyage et/ou le démaquillage de la peau et/ou des cheveux, le soin de la peau et/ou des cheveux, pour la protection de la peau contre le soleil, pour le maquillage de la peau.
